# EUROPEAN PATENT APPLICATION

(11) **EP 2 543 346 A1**
(43) Date of publication of application: **09.01.2013**
(21) Application number: 12187494.5
(22) Date of filing: 12.01.2007
(51) Int. Cl.: A61F 2/90

(54) **Hybrid stent**

(30) Priority: 13.01.2006 US 331639
(62) Divisional of application: 07700481.0
(71) Applicant: MEDINOL LTD., 61581 Tel Aviv (IL)
(72) Inventor: Richter, Jacob, 46920 Arsuf (IL)
(74) Representative: Kuhnen & Wacker

(57) **Abstract**

A stent (1) is provided with a series of short pieces or sections (2) connected together by a bioresorbable polymer (3). The stent sections are designed to separate or articulate with time as the polymer biodegrades. The time of separation can be controlled by the characteristics of the bioresorbable polymer to allow the stent to be buried in neo-intima. By using a tube made of a bioresorbable polymer, the continuous covering of the tubing may inhibit embolization in the first few weeks after stent implantation within the walls of a vessel and timing for removal of the tube through formulation of the bioresorbable polymer can be controlled to occur when embolization is no longer a risk. When the detachment of the stent pieces or sections occurs, they are fixedly secured within the vessel and each is able to flex with the vessel independently of the other stent segments.

## Description

### CROSS-REFERENCE TO RELATED APPLICATIONS

This application is a continuation-in-part of co-pending U.S. patent application 10/860,735, filed June 3, 2004, which is a continuation-in part of co-pending U.S. patent application 10/116,159 filed on April 5, 2002, which is a continuation of U.S. patent application 09/204,830 filed on December 3, 1998, now abandoned.

### FIELD OF THE INVENTION

The invention relates generally to stents, which are endoprostheses implanted into vessels within the body, such as a blood vessels, to support and hold open the vessels, or to secure and support other endoprostheses in vessels.

### BACKGROUND OF THE INVENTION

Various stents are known in the art. Typically stents are generally tubular in shape, and are expandable from a relatively small, unexpanded diameter to a larger, expanded diameter. For implantation, the stent is typically mounted on the end of a catheter, with the stent being held on the catheter at its relatively small, unexpanded diameter. Using a catheter, the unexpanded stent is directed through the lumen to the intended implantation site. Once the stent is at the intended implantation site, it is expanded, typically either by an internal force, for example by inflating a balloon on the inside of the stent, or by allowing the stent to self-expand, for example by removing a sleeve from around a self-expanding stent, allowing the stent to expand outwardly. In either case, the expanded stent resists the tendency of the vessel to narrow, thereby maintaining the vessel's patency.

Some examples of patents relating to stents include U.S. Patent No. 4,733,665 to Palmaz; U.S. Patent No. 4,800,882 and 5,282,824 to Gianturco; U.S. Patent Nos. 4,856,516 and 5,116,365 to Hillstead; U.S. Patent Nos. 4,886,062 and 4,969,458 to Wiktor; U.S. Patent No. 5,019,090 to Pinchuk; U.S. Patent No. 5,102,417 to Palmaz and Schatz; U.S. Patent No. 5,104,404 to Wolff; U.S. Patent No. 5,161,547 to Tower; U.S. Patent No. 5,383,892 to Cardon et al.; U.S. Patent No. 5,449,373 to Pinchasik et al.; and U.S. Patent No. 5,733,303 to Israel et al.

One object of prior stent designs has been to insure that the stent has sufficient radial strength when it is expanded so that it can sufficiently support the lumen. Stents with high radial strength, however, tend also to have a higher longitudinal rigidity than the vessel in which it is implanted. When the stent has a higher longitudinal rigidity than the vessel in which it is implanted, increased trauma to the vessel may occur at the ends of the stent, due to stress concentrations on account of the mismatch in compliance between the stented and un-stented sections of the vessel.

### SUMMARY OF THE INVENTION

An object of the invention is to provide a stent that more closely matches the compliance of the vessel in which it is implanted, with relatively little or no sacrifice in radial strength, even when the stent is made very long.

In accordance with one embodiment of the invention, a stent is provided with specific "designated detachment" points, such that after the stent is deployed, and during the motion of the vessel, the stress applied on the stent will cause the stent to separate at these designated detachment points. When the designated detachment points are arranged completely around the circumference of the stent, creating a circumferential "designated detachment" zone, the detachment at the designated detachment points separates the stent into two or more separate sections or pieces (hereafter "sections"), each able to move with the vessel independently of one another. Because each separate section can move independently, a series of separate sections can achieve greater compliance between the stented and un-stented sections of the vessel than a longer stent product, and thereby reduce stress on the vessel wall. The short sections that would potentially be unstable in the vessel and would tend to topple over, are secured against toppling by a longitudinal structure at the time of implant that may be bio absorbed or separated with time. This separation into short sections would occur preferably after the stent struts would have been covered with neo-intima that will secure them in place.

The stent of the invention is preferably designed such that after detachment, the ends of each section created thereby are relatively smooth, so that they do not injure the vessel wall. Also, the stent is preferably configured such that the combination of separate sections has sufficient radial strength after detachment, and results in little or no significant reduction in the stent's resistance to compression.

The stent would preferably be designed such that detachment occurs only after a period of time following implantation, so that the stent will already be buried under neointima at the time of detachment. Thus, the separate sections remaining after detachment will be held in place by the neointima and will not move relative to the lumen, i.e., they will not "telescope" into one another, and they will not move away from one another, creating unsupported gaps.

A variety of mechanisms may be used to accomplish the detachment. For example, the stent may be provided at certain points or zones along its length with components having a cross-sectional area sufficiently low so that the sections will detach from each other preferentially under the stress placed on the stent after implantation. Alternatively or additionally, the stent may be provided with certain points or zones along its length with components and/or material that is sufficiently weaker than elsewhere in the stent so that the sections will detach preferentially under the stress placed on the stent after implantation. Alternatively or additionally, the stent may be designed such that it has a lower number of components, or struts, at the designated detachment zones, so that each such component bears more load than components elsewhere in the stent. These components are configured to separate under the increased loads they bear when the stent is repeatedly stressed after implantation.

The factors contributing to detachment may be applied individually or in combination. For example, the designated detachment struts may have low cross-sectional areas and also may be formed of weaker material, or the designated detachment zones may have a reduced number of components, with or without the components having low cross-sectional areas and/or being formed of weaker material.

Another mechanism of detachment is the use of bioresorbable or biodegradable material. A bioresorbable or biodegradable material is a material that is absorbed into or degraded by the body by active or passive processes. Similarly, certain biocompatible materials are "resorbed" by the body, that is, these materials are readily colonized by living cells so that they become a permanent part of the body. Such materials are also referred to herein as bioresorbable or durable polymers. When either type of material is referred to in the foregoing description, it is meant to apply to both bioresorbable and biodegradable materials.

The present invention relates to a series of otherwise separate pieces or sections which are interconnected to form a stent of a desired length by using a longitudinal structure made of bioresorbable material. The original stent structure will thus eventually disintegrate to leave a series of its constituent short sections or pieces, resulting in a longitudinal flexibility and extendibility closer to that of a native vessel. It is desirable to design the longitudinal structure such that it would promote the growth of neo-intima that will fixate the short sections or pieces into the desired position before the longitudinal structure is absorbed or degraded, and thus prevent movement of those sections thereafter.

The longitudinal structure of the bioresorbable material may be porous or it may be formed as a tube with fenestrations or a series of fibers with spaces between them, to promote faster growth of neo-intima that will cover the stents and secure them in position before degradation of the structure. Fenestrations may also promote better stabilization of the stent before degradation of the bioresorbable material. The shape of fenestration can be made in any desired size, shape or quantity.

It will be appreciated that the separation between sections can be controlled by the characteristics of the bioresorbable material. Preferably, separation occurs after the stent is buried in neo-intima and the short sections are stabilized.

A stent utilizing bioresorbable material may contain separate sections or pieces that are shorter than could ordinarily function as an individual stent, because they are stabilized at the time of deployment by the longitudinal structure in which they are embedded and then retained by the neo-intimal growth. The stent may be of any desired design. The stent may be made for implanting by either balloon expansion or self expansion and made of any desired stable material.

The present invention allows the bioresorbable material to be manufactured at any length. In one embodiment, the stent in the supporting structure may be manufactured as a long tube and then cut to customize the length of the implanted stent for a particular patient.

Another method of achieving the same result of a high radial resistance but very low resistance to longitudinal bending, may be a stent that has separate metal sections held together by a very soft longitudinal structure made from a durable polymer materials.

### BRIEF DESCRIPTION OF THE DRAWINGS

- Figure 1: shows a schematic diagram of a stent, generally in the form of a cylinder, having designated detachment zones between sections;
- Figure 2: shows a schematic diagram of the stent of Figure 1 after detachment, in which the stent has separated into a series of shorter sections;
- Figure 3: shows a flat layout of a stent pattern in which the components in the designated detachment zones have a cross-sectional area that is sufficiently low so that the stent will separate into its constituent sections or pieces as a result of the stress placed on the stent after implantation;
- Figure 4: shows a flat layout of the stent pattern of Figure 3, after separation has occurred at the designated detachment zones; and
- Figure 5: shows a flat layout of a stent pattern in which the stent has a lower number of detachment components at the designated detachment zones.
- Figure 6: illustrates a side view layout of a stent as separate circumferential stent pieces embedded in a bioresorbable material.
- Figure 7: illustrates a side view layout of a series of short sections embedded in a bioresorbable material.
- Figure 8: illustrates a side view layout of a stent made as a series of circumferential pieces or members embedded in a bioresorbable polymer tubing with fenestrations.
- FIGURE 9: illustrates a photomicrograph of stent members connected by a very porous polymeric structure.

### DETAILED DESCRIPTION OF THE DRAWINGS

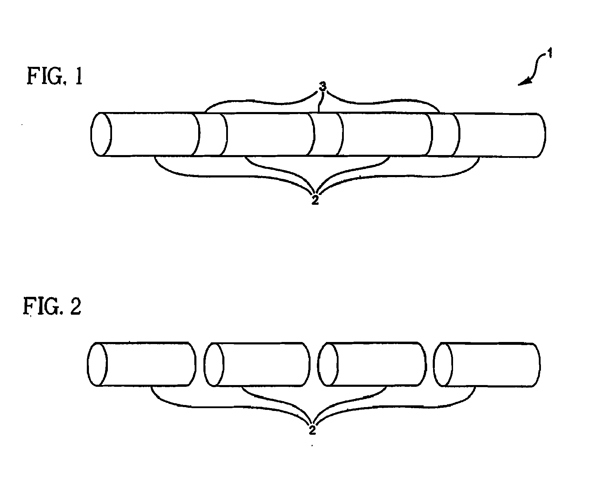

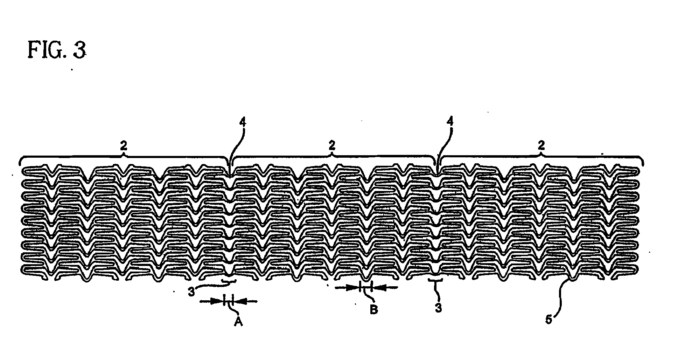

Figure 1 shows a conceptualized schematic diagram of a stent 1, generally in the form of a cylinder. The stent 1 comprises a series of separable sections 2 spaced apart by designated detachment zones 3. The designated detachment zones 3 comprise one or more designated detachment components or struts (see Figures 3 through 5).

The designated detachment zones 3 are designed such that the designated detachment components fracture or otherwise create a separation under repeated stress placed on the stent 1 after implantation. When all of the designated detachment struts around the circumference of the stent in a particular designated detachment zone 3 separate, the stent is itself separated into a series of independent sections 2, as shown in Figure 2. The designated detachment zones 3 may be designed such that detachment does not occur until some time has passed after implantation, so that the resulting separate sections 2 will already be buried under neointima at the time of detachment and therefore will not move relative to the lumen.

Persons of ordinary skill in the art will appreciate that the basic geometry of the sections 2 may take any suitable form, and that they may be formed of any suitable material. Examples of suitable structures for the sections 2 include, but are not limited to, those shown in U.S. Patent No. 5,733,303 to Israel et al., or as forming part of the NIR™ stent manufactured by Medinol Ltd. The disclosure of this patent is hereby expressly incorporated by reference into this application. Other examples of suitable structures for the sections 2, include but are not limited to, those shown in U.S.Patents Nos. 6,723,119 and 6,709,453 to Pinchasik et al., or forming part of the NIRflex™ stent, which is also manufactured by Medinol Ltd. The disclosures of these patents are also expressly incorporated by reference into this application. Other suitable stent structures may be used in the present invention and their identification is readily known to the skilled artisan based upon the teaching of the present invention.

Figure 3 shows a flat layout of a stent pattern comprising sections 2 separated by designated detachment zones 3. As here embodied, the stent pattern corresponds generally to one described in U.S. Patent No. 5,733,303, except that sections 2 are joined to each other by the designated detachment components or struts (indicated at 4) in the designated detachment zones 3.

In this embodiment, each of the designated detachment struts 4 has a reduced cross-sectional area (relative to the balance of the pattern) that is sufficiently low to allow separation at the designated detachment struts 4 under the stress placed on the stent after implantation. The amount of reduction of the cross-section of the detachment struts 4 as compared to, for example, the components labeled by reference numeral 5 in the sections 2, may be, for example, on the order of tens of percents. For example, the detachment struts 4 may be 25% to 75% thinner or narrower in the circumferential direction of the stent than the components 5.

These designated detachment struts 4 may additionally or alternatively be made of a weaker material, in order to ensure appropriate separation or fracture. The weaker material, in terms of tensile strength, may be provided either in the stock material used to form the designated detachment struts 4, or by treating the designated detachment struts 4 (or the designated detachment zones 3) after the stent has been produced, such that the treatment weakens the material of the designated detachment struts 4.

One approach for weakening the designated detachment struts is to form the entire stent of NiTi and then to treat the designated detachment struts to be Martensitic while the remaining components will be in the Austenitic phase. Another approach is to make the stent of stainless steel and hardening all but the designated detachment zones, which would be annealed.

In addition to the reduction in cross-section, the remaining geometry of the designated detachment struts may be selected to achieve the desired results. As shown in Figure 3, the width A of the row of designated detachment struts 4 may be narrower than the width of a corresponding row of components in the sections 2, for example the width B of the row of components labeled by reference numeral 5. This reduced width at the designated detachment zones 3 helps to ensure detachment at the designated detachment zones 3 under repeated longitudinal bending. Also, the designated detachment struts 4 may be made sufficiently short to reduce the length of the free ends after separation, so as not to leave long, hanging ends after detachment and thereby minimize the chance for tissue injury. For example, the length of the designated detachment struts 4 is shorter than the length of the components 5.

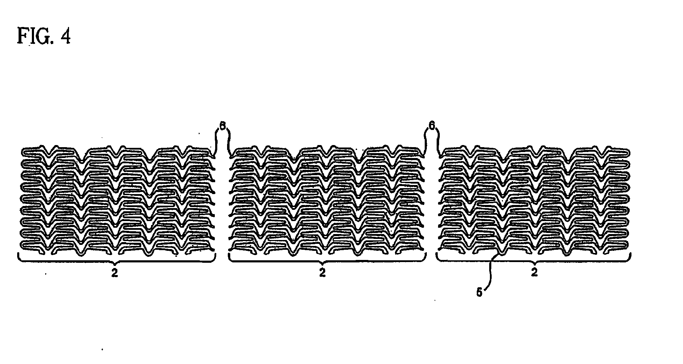

Figure 4 shows a flat layout of the stent pattern of Figure 3 after detachment has occurred at the designated detachment zones 3. As shown in Figure 4, the stent after detachment comprises a series of separated and independent sections 2. As also seen in Figure 4, because the designated detachment struts 4 were short, the length of the free ends 6 after separation is kept to a minimum.

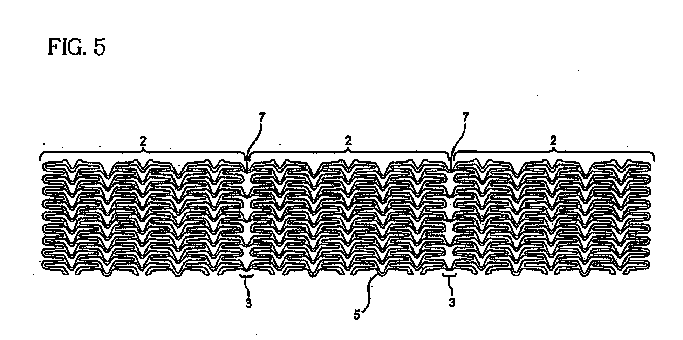

Figure 5 shows an alternative design in which the designated detachment zones 3 include fewer detachment components (here indicated at 7) around the circumference of the stent. In the embodiment shown in Figure 5, each designated detachment zone 3 has five designated detachment struts 7 around the circumference of the stent (as compared with nine in Figure 3). Of course, different numbers of designated detachment struts and stent segment components may be used, without departing from the general concept of the invention.

The designated detachment struts 7 are configured such that they detach under the loads they bear on account of the stress placed on the stent after implantation. As shown in Figure 5, the designated detachment struts 7 may also have a reduced cross-sectional area. Also, as with the designated detachment struts in other embodiments, the designated detachment struts 7 may additionally be formed of weaker material, or the designated detachment struts 7 or zones 3 may be treated to make the material weaker after production of the stent.

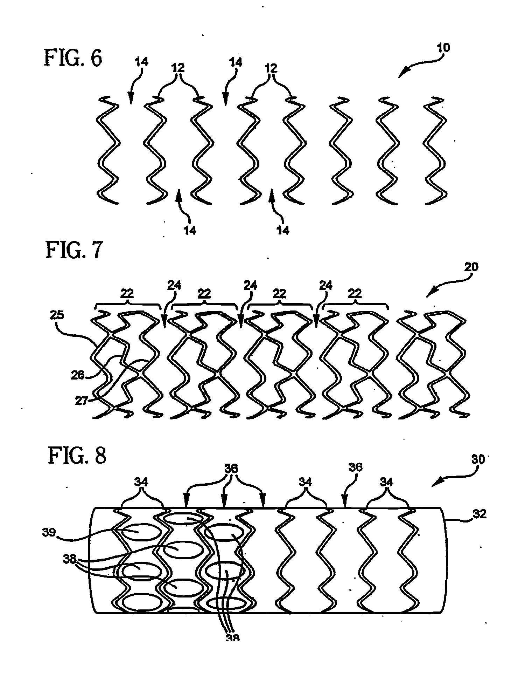

Figure 6 illustrates one example of using a bioresorbable material. Stent 10 of Figure 6 comprises a series of generally circumferentially extending pieces 12 which are interconnected by a bioresorbable material. The bioresorbable material may be placed within the spaces 14 between the pieces 12, or the latter may be embedded in the bioresorbable material. Alternatively, the pieces 12 may be coated with the bioresorbable material, or connected by fibers of bioresorbable material or undergo any processing method known to one skilled in the art to apply the bioresorbable material to the constituent pieces or sections. The coating thickness of the polymer on the circumferential pieces or extent to how deep the pieces are embedded in the polymer may be varied and may control the timing of detachment of the constituent pieces.

Any stent design may be utilized with the bioresorbable material in the manner taught by the present invention. In this example the circumferential pieces can be any structure which provides a stored length to allow radial expansion such as single sinusoidal members. However, it should be understood that the invention is not limited to any particular structure or design. For example, the circumferential pieces can be of the same design throughout the stent or they may be of different designs depending on their intended use or deployment. Thus, the invention also permits a stent design in which various circumferential pieces can have different structural or other characteristics to vary certain desired properties over the length of the stent. For example, the end pieces of the stent can be more rigid (e.g., after expansion) than those in the middle of the start.

This example is only given as an illustration and is not meant to limit the scope of the invention. Any stent design can be used in the present invention. The individual design of each circumferential piece can be uniform or not, depending on the stent application.

Upon deployment in a vessel to cover a long lesion, the bioresorbable material connects the series of constituent pieces or sections together until a time when the material degrades and the constituent pieces or sections will have separated from each other. The individual sections now can articulate, move, or flex independently of each other as the vessel flexes or stretches, to allow natural movement of the vessel wall. Thus, in this embodiment of the invention, the stent bends between sections or pieces according to the natural curvature of the vessel wall.

The separation time using the bioresorbable material as the longitudinal structure of the stent can be controlled by the characteristics of the bioresorbable material. Preferably, the stent sections will have been buried in a layer of neointima and the short sections stabilized before the bioresorbable material is resorbed.

There are several advantages of using the bioresorbable material. As previously shown, there is an advantage of controlling the release of the constituent pieces or sections by modifying or choosing the characteristics of the bioresorbable material.

Additionally, the bioresorbable material does not obscure radiographs or MRI/CT scans, which allows for more accurate evaluation during the healing process. Another advantage of using the bioresorbable material is that the continuous covering provided by the bioresorbable material after the stent is deployed in a vessel is believed to inhibit or decrease the risk of embolization. Another advantage is the prevention of "stent jail" phenomenon, or the complication of tracking into side branches covered by the stent.

The depletion of the bioresorbable material covering can be controlled by modification or choosing characteristics of the bioresorbable material to allow degradation at a time about when the sections are fixated in the vessel wall and embolization is no longer a risk. Examples of altering the biodegradable or bioresorbable material by modification or changing the material characteristics of the polymer are described below as to the extent and speed a material can degrade. It should be understood that these modifications and characteristics are merely examples and are not meant to limit the invention to such embodiments.

The sections can be made of any material with desirable characteristics for balloon expandable stent or self-expandable stenting. For example, materials of this type can include but are not limited to, stainless steel, nitinol, cobalt chromium or any alloy meeting at least as a minimum the physical property characteristics that these materials exhibit.

The material of the bioresorbable material can be any material that is either readily degraded by the body and can be naturally metabolized, or can be resorbed into the body. In particular, bioresorbable materials are selected from light and porous materials which are readily colonized by living tissues to become a permanent part of the body. For example, the bioresorbable material can be, but is not limited to, a bioresorbable polymer. For example, any bioresorbable polymer can be used with the present invention, such as polyesters, polyanhydrides, polyorthoesters, polyphosphazenes, and any of their combinations in blends or as copolymers. Other usable bioresorbable polymers can include polyglycolide, polylactide, polycaprolactone, polydioxanone, poly(lactide-co-glycolide), polyhydroxybutyrate, polyhydroxyvalerate, trimethylene carbonate, and any blends and copolymers of the above polymers.

Synthetic condensation polymers, as compared to addition type polymers, are generally biodegradable to different extents depending on chain coupling. For example, the following types of polymers biodegrade to different extents (polyesters biodegrade to a greater extent than polyethers, polyethers biodegrade to a greater extent than polyamides, and polyamides biodegrade to a greater extent than polyurethanes). Morphology is also an important consideration for biodegradation. Amorphous polymers biodegrade better than crystalline polymers. Molecular weight of the polymer is also important. Generally, lower molecular weight polymers biodegrade better than higher molecular weight polymers. Also, hydrophilic polymers biodegrade faster than hydrophobic polymers. There are several different types of degradation that can occur in the environment. These include, but are not limited to, biodegradation, photodegradation, oxidation, and hydrolysis. Often, these terms are combined together and called biodegradation. However, most chemists and biologists consider the above processes to be separate and distinct. Biodegradation alone involves enzymatically promoted break down of the polymer caused by living organisms.

As a further advantage of the invention, the structure may be embedded with drug that will inhibit or decrease cell proliferation or will reduce restenosis in any way. Further, a material containing a longitudinal structure of fibers provides a continuous structure with small inter-fiber distance and provides a more uniform elution bed as a matrix for eluting drug. In one embodiment, the constituent pieces or sections may be treated to have active or passive surface components such as drugs that will be advantageous for the longer time after those sections are exposed by bioresorption of the longitudinal structure.

Figure 7 illustrates a stent 20 that is another example of the present invention. Instead of being made of a series of circumferential pieces or members as in Figure 6, this embodiment contains short sections indicated at 22. Again, as with Figure 6, these stent sections 22 can be any design and are not limited to the embodiment shown in Figure 7. Stent 20, as with the stent of Figure 6, can have identical short stent sections or not depending on the application of the stent.

The stent sections may be made of any suitable material and may form any acceptable design. The stent may be balloon expandable or self-expandable.

Example designs are described in U.S. Patent No. 6,723,119, which is incorporated herein in toto, by reference. Another example design is the NIRflex stent which is manufactured by Medinol, Ltd. One such example is shown in Figure 7. This design criteria can result in short sections which provide longitudinal flexibility and radial support to the stented portion of the vessel.

The bioresorbable material can be disposed within interstices 24 and/or embedded throughout the stent segments. The bioresorbable material may cover the entire exterior or only a portion of the stent segments or fully envelop all the segments.

Figure 8 illustrates another example of the present invention in the form of stent 30 having a bio-resorbable material 32 in the form of a tube. As here embodied, the tube interconnects circumferential pieces (or members) 34 with the bio-resorbable material filling interstices 36. The pieces 34 illustrated in figure 8 are single sinusoidal members, but can be of any design or multitude of designs as previous discussed.

Stent 30 may also include fenestrations 38. Fenestrations can be any shape desired and can be uniformly designed such as the formation of a porous material for example, or individually designed. The non-continuous layered material can also be formed in other ways such as a collection of bioresorbable fibers connecting the pieces. Fenestration of the bioresorbable cover may promote faster growth of neo-intima and stabilization of the short segments before integration or degradation of the bioresorbable material. The present invention allows the bioresorbable material to be manufactured at any length and then cut in any desired length for individual functioning stents to assist manufacturing the stent. For example, in the case of bioresorbable polymer tubing illustrated in Figure 8, the tubing can be extruded at any length and then cut to customize the stent, either by the manufacturer or by the user.

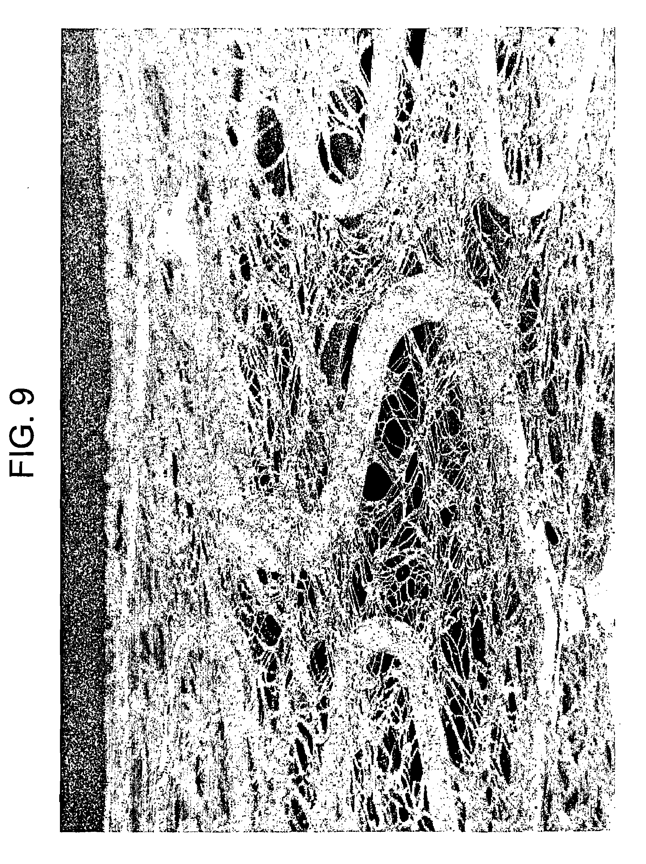

FIGURE 9 illustrates a photomicrograph of stent members connected by a porous longitudinal structure along a longitudinal axis of the stent. This longitudinal structure may or may not be polymeric, depending on the properties desired. In one embodiment, the longitudinal structure is a porous fiber mesh like a durable polymer. One example of such a material includes, but is not limited to, polytetrafluoroethylene (ePTFE). The longitudinal structure, among other functions, provides longitudinal flexibility to the stent members. The stent members may or may not be a metallic structure, depending on the desired properties. The longitudinal structure also may provide a continuous structure having small inter-fiber distances and forming a matrix. This matrix may be used for eluting a drug and would provide a more uniform elution bed over conventional methods.

It may be advantageous to employ a light and porous polymeric material. For example, a fibrous material may be constructed so that the fibers provide a longitudinal structure thereby enhancing the overall flexibility of the stent device Such a material may be applied to a stent or stent pieces in a continuous or non-continuous manner depending upon the particular needs of the structure contemplated. The material may be any polymeric material. An example of such a material is expanded polytetrafluoroethylene (ePTFE), but is not limited to this material. The polymeric material can form a porous fiber mesh that is a durable polymer. The longitudinal structure serves at least two functions. First, the longitudinal structure is more longitudinally flexible than a conventional metallic structure. Second, the polymeric material is a continuous structure with small inter-fiber distance and can be used as a matrix for eluting drug that would provide a more uniform elution bed.

It should be understood that the above description is only representative of illustrative examples of embodiments. For the reader's convenience, the above description has focused on a representative sample of possible embodiments, a sample that teaches the principles of the invention. Other embodiments may result from a different combination of portions of different embodiments. The description has not attempted to exhaustively enumerate all possible variations.

Again, the embodiments described herein are examples only, as other variations are within the scope of the invention as defined by the appended claims.

## Claims

1. A stent comprising:
a) a structure in the form of a tube having one or more interconnected members; and
b) a durable polymer material having preselected areas of fenestration, said fenestrated areas occurring within the structure.

2. The stent according to claim 1, wherein the one or more interconnected members are one or more sinusoidal members, wherein said polymer material between said fenestrated areas interconnects adjacent sinusoidal members of said structure.

3. The stent according to claim 2, wherein said sinusoidal members are vertical sinusoidal members.

4. The stent according to claim 2, wherein said sinusoidal members are generally circumferentially extending sinusoidal members.

5. The stent according to claim anyone of the preceding claims, wherein the fenestrated areas are oval in shape.

6. The stent of claim 5, wherein the oval-shaped fenestrated areas are positioned between members of the structure.

7. The stent according to any one of the preceding claims, wherein the polymer material is polyurethane or ePTFE.

8. The stent according to any one of the preceding claims, wherein the polymer material provides longitudinal flexibility to the stent.

9. The stent according to any one of the preceding claims, wherein the polymer material provides structural support to the stent.

10. The stent according to any one of the preceding claims, wherein the polymer material interconnects adjacent members of the structure in the longitudinal direction.

11. The stent according to any one of the preceding claims, wherein the polymer material is affixed to the stent by electro-spinning.

12. The stent according to any one of the preceding claims, wherein the polymer material extends through the entire length of the stent.

13. The stent of any one of the preceding claims, wherein the members of the structure are embedded in the polymer.

14. The stent according to any one of the preceding claims, further comprising a drug coating.

15. The stent according any one of the preceding claims, wherein the structure is a metallic structure.

16. The stent according to claim 15, wherein the metallic structure comprises an amorphous metal alloy.

17. The stent according to claim 16, wherein said amorphous metal alloy comprises an element selected from the group consisting of silicon, boron, and phosphorous; and/or
is an iron-based alloy containing Fe, Cr, B, and P; and/or
contains silicon; and/or
comprises a Fe Cr B P alloy.

18. The stent according to any one of claims 2 to 17, wherein said fenestrated areas occur between each adjacent sinusoidal members.

19. The stent according to any one of claims 2 to 18 wherein the polymer material interconnects adjacent sinusoidal members in the longitudinal direction.
